# EUROPEAN PATENT SPECIFICATION

(11) **EP 4 456 875 B1**
(45) Date of publication and mention of the grant of the patent: **15.10.2025**
(21) Application number: 22844563.1
(22) Date of filing: 30.12.2022
(51) Int. Cl.: A61K 9/08, A61K 31/454, A61K 47/10, A61K 47/26

(54) **ORAL SOLUTION COMPRISING LENALIDOMIDE**
ORALE LÖSUNG MIT LENALIDOMID
SOLUTION ORALE COMPRENANT DE LA LÉNALIDOMIDE

(30) Priority: 31.12.2021 EP 21386083
(43) Date of publication of application: 06.11.2024
(73) Proprietor: a Fine House S.A., 19441 Koropi (GR)
(72) Inventor: LIOLIOS, Georgios, 15232 Chalandri Attica (GR)
(74) Representative: Roukounas, Dimitrios
(86) International application number: PCT/EP2022/088099
(87) International publication number: WO 2023/126530

(56) References cited:
- US-A1- 2012 283 292
- US-A1- 2016 194 301

## Description

### TECHNICAL FIELD

The present invention relates to oral pharmaceutical solutions comprising lenalidomide or a pharmaceutically acceptable lenalidomide salt.

### BACKGROUND OF THE INVENTION

Lenalidomide is an analog of thalidomide with antineoplastic activity. Lenalidomide inhibits TNF-alpha production, stimulates T cells, reduces serum levels of the cytokines vascular endothelial growth factor (VEGF) and basic fibroblast growth factor (bFGF), and inhibits angiogenesis. It also promotes G1 cell cycle arrest and apoptosis of malignant cells.

Lenalidomide is a racemic mixture, which has the chemical name 1-oxo-(2,6-dioxopiperidin-3-yl)-4-aminoisoindoline and the following structural formula:

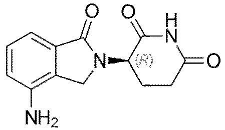

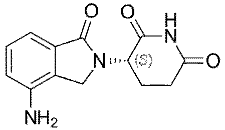

Lenalidomide and its process of manufacture were first disclosed in U.S. patent no. 5,635,517.

Lenalidomide is soluble in organic solvent/water mixtures and buffered aqueous solutions and exhibits the highest solubility of 18.0 mg/ml in a 0.1 N HCl aqueous solution (i.e. an aqueous solution with pH values around 1). Solubility in basic solutions decreases to about 0.4 to 0.5 mg/ml.

Besides pH, lenalidomide solubility is influenced by its crystalline form. As disclosed in US patent 7,465,800, polymorph A is characterized as the most soluble crystalline form with a solubility of 6.2 mg/ml in an aqueous HCl solution at pH 1.8, containing 1% sodium lauryl sulfate. Polymorphs B and E exhibit solubility of 5.8 mg/ml and 4.7 mg/ml, respectively, under the same experimental conditions.

Currently, lenalidomide is approved in Europe as monotherapy for the maintenance treatment of adult patients with newly diagnosed multiple myeloma who have undergone autologous stem cell transplantation or as combination therapy with dexamethasone, or bortezomib and dexamethasone, or melphalan and prednisone for the treatment of adult patients with previously untreated multiple myeloma who are not eligible for transplant. It is also indicated as monotherapy for the treatment of adult patients with transfusion-dependent anaemia due to low- or intermediate-1-risk myelodysplastic syndromes associated with an isolated deletion 5q cytogenetic abnormality when other therapeutic options are insufficient or inadequate. In addition, it is indicated for the treatment of adult patients with relapsed or refractory mantle cell lymphoma. Furthermore, lenalidomide in combination with rituximab (anti-CD20 antibody) is indicated for the treatment of adult patients with previously treated follicular lymphoma

The commercial lenalidomide capsules are marketed under the trade names Revlimid^{®} and Ladevina^{®}. These capsules are marketed in strengths of 2.5 mg, 5 mg, 7.5 mg, 10 mg, 15 mg, 20 mg and 25 mg of lenalidomide free base.

Although oral solid dosage forms such as tablets and capsules are very popular mainly due to ease of management, for certain users (e.g. children and the elderly) these forms are not necessarily a convenient option, especially due to difficulty in swallowing these forms. This lack of convenience results in high incidence of non-compliance and ineffective therapy.

Moreover, the Patient Information Leaflet (PIL) of Revlimid^{®} tablets discloses a dosing scheme according to which the dosage is adjusted based on the medical condition of the patient, response to treatment, and lab test results. The concept of individualised, tailored treatment sets as prerequisite pharmaceutical forms, that enable dose fractioning, such as oral solutions.

Hence, as a means of both ensuring flexibility in dose titration and advanced patient compliance, the development of oral liquid dosage forms of lenalidomide, which can allow optimal dose selection is definitely an existing need.

Patent application WO 1998/03502 discloses in Example 23 a 0.2% injection or infusion solution comprising lenalidomide, sodium chloride, phosphate buffer pH 7.4 and demineralized water.

However, the desire for the development of an oral liquid dosage form that comprises lenalidomide is complicated by the fact that lenalidomide and its pharmaceutically acceptable salts are unstable in liquid environment under acidic and basic hydrolytic conditions as well as under oxidative conditions.

The present invention overcomes the problems of the prior art and provides an oral pharmaceutical solution, comprising lenalidomide or a pharmaceutically acceptable salt of lenalidomide, which exhibits excellent stability and extended lifetime.

### SUMMARY OF THE INVENTION

The present invention is directed to an oral pharmaceutical solution comprising lenalidomide or a pharmaceutically acceptable salt thereof in association with a pharmaceutically acceptable aqueous carrier. according to present claims.

The oral pharmaceutical solution according to the invention comprises lenalidomide or a pharmaceutically acceptable salt thereof as active ingredient and a pharmaceutically acceptable carrier comprising a glycol selected from propylene glycol, polyethylene glycol having an average molecular weight from 150 to 1500, or mixtures thereof; a polyol selected from glycerol, sorbitol, mannitol, maltitol, xylitol, erythritol, isomalt, lactitol, polyvinyl alcohol, or mixtures thereof; and water, wherein the pH of the solution is from 1.0 to 3.0.

The oral pharmaceutical solution according to the present invention provides the best alternative over conventional capsule or film coated tablet dosage forms. Apart from achieving better patient compliance, the oral solution of the present invention offers unique advantages such as more reproducible bioavailability and an option of a flexible dosing regimen.

### DETAILED DESCRIPTION OF THE INVENTION

The present invention provides an oral pharmaceutical solution comprising lenalidomide or a pharmaceutically acceptable lenalidomide salt as active ingredient. Numerous combinations of cosolvents and surfactants such as polysorbate 20, 40, 60 or 80, povidone K25, K30, K29/32, 90F, sodium lauryl sulfate, hydroxypropyl cellulose, hydroxypropyl methylcellulose, poloxamer 20, 40, 60, fructose, amino acids, sucrose and glucose, were evaluated by the present inventors but did not provide the desired solubility of lenalidomide and/or stability of the oral solution.

A composition was prepared according to example 23 of WO 1998/03502. The solution contained 5.0 g lenalidomide, 22.5 g sodium chloride, 300.0 g phosphate buffer with pH 7.4 and demineralized water. The pH of the final composition was 7.4. However, precipitation was observed within minutes of the preparation of the composition.

Similarly, attempts to prepare liquid compositions of lenalidomide in any one of sorbitol, glycerol, PEG-400, propylene glycol, PEG-1450 (10% solution), maltitol or purified water, wherein the pH of the compositions was from 5.0 to 8.0, were unsuccessful, due to precipitation that was observed in all trials within minutes of the preparation of the compositions.

Similar results (i.e. precipitation) were obtained a few minutes after lenalidomide was dissolved in an aqueous buffer at pH between 1.0 and 3.5.

Likewise, attempts to prepare an oral solution by combining lenalidomide with any one of arginine, leucine, povidone K25, sodium lauryl sulfate, hydroxypropyl methylcellulose, poloxamer 20, in an aqueous buffer at pH 2.0 or pH 2.5 led to the formation of precipitate within minutes of the preparation of the compositions.

After intensive testing it was surprisingly found that when at least one polyol and at least one glycol is added to a lenalidomide aqueous solution, wherein the pH has been adjusted at low acidic values, it is possible to manufacture an oral solution instead of a suspension of lenalidomide or a salt thereof having excellent physicochemical stability.

The oral pharmaceutical solution according to the invention comprises lenalidomide or a pharmaceutically acceptable salt thereof as active ingredient and a pharmaceutically acceptable carrier comprising a) a glycol, b) a polyol, c) water, wherein the pH of the solution is from 1.0 to 3.0.

As used throughout this description and claims, a "glycol" may be propylene glycol, polyethylene glycol or any other pharmaceutically acceptable polyalkylene glycol product such as those known in the art as the "PEG" series, or mixtures thereof.

Preferably the glycol is selected from propylene glycol, polyethylene glycol having an average molecular weight from 150 to 1500, or mixtures thereof.

More preferably, the polyethylene glycol has an average molecular weight of 200 (also known as polyethylene glycol 200, or PEG 200), or of 300 (also known as polyethylene glycol 300, or PEG 300), or of 400 (also known as polyethylene glycol 400, or PEG 400), or of 600 (also known as polyethylene glycol 600, or PEG 600), or of 800 (also known as polyethylene glycol 800, or PEG 800) or of 1450 (also known as polyethylene glycol 1450, or PEG 1450). Even more preferably, the polyethylene glycol has an average molecular weight of 400.

As used throughout this description and claims, the term "polyol" (polyhydric alcohol) refers to pharmaceutical excipients containing multiple hydroxyl groups. Although the term "polyol" includes sugar alcohols, it does not include sugars, i.e. carbohydrates, such as sucrose, glucose, dextrose, fructose and galactose. Typical examples of suitable polyols according to the invention are sugar alcohols such as, mannitol, maltitol, glycerol, sorbitol, xylitol, erythritol, isomalt and lactitol, as well as polyvinyl alcohol.

Preferably, the polyol is selected from glycerol, sorbitol, mannitol, maltitol, xylitol, erythritol, isomalt, lactitol, polyvinyl alcohol, or mixtures thereof. More preferably, the polyol is selected from glycerol, sorbitol, mannitol, maltitol, xylitol, or mixtures thereof. Even more preferably, the polyol is glycerol.

Any pharmaceutically acceptable system which acts as a buffer in the pH region of the invention can be used in the oral pharmaceutical solution according to the invention. Examples of buffering agents include but are not limited to ascorbic acid, acetic acid, tartaric acid, citric acid monohydrate, sodium citrate, potassium citrate, acetic acid, sodium acetate, sodium hydrogen phosphate, sodium dihydrogen phosphate, calcium hydrogen phosphate, calcium dihydrogen phosphate, or mixtures thereof.

The oral pharmaceutical solution according to the invention may comprise lenalidomide or any pharmaceutically acceptable salt of lenalidomide such as hydrochloride, hydrobromide, methane sulfonate, ethane sulfonate, benzene sulfonate and p-toluenesulfonate. Preferably, the oral pharmaceutical solution according to the invention comprises lenalidomide.

Preferably, the oral pharmaceutical solution according to the invention comprises from 2 mg/ml to 10 mg/ml of lenalidomide or a pharmaceutically acceptable salt of lenalidomide. More preferably, the solution comprises from 4 mg/ml to 8 mg/ml of lenalidomide or a pharmaceutically acceptable salt of lenalidomide. Even more preferably, the solution comprises from 5 mg/ml to 7 mg/ml of lenalidomide or a pharmaceutically acceptable salt of lenalidomide.

Preferably, the glycol concentration, in the oral pharmaceutical solution according to the invention is from 200 to 600 mg/ml. More preferably, the glycol concentration is from 250 mg/ml to 550 mg/ml. Even more preferably, the glycol concentration is from 300 mg/ml to 500 mg/ml.

Preferably, the polyol concentration in the oral pharmaceutical solution according to the invention is from 50 to 400 mg/ml. More preferably, the polyol concentration is from 100 mg/ml to 350 mg/ml. Even more preferably, the polyol concentration is from 100 mg/ml to 300 mg/ml.

The term "mg/ml" used in the present description and claims means mg of the active ingredient or of the excipient(s) per 1 ml of the oral pharmaceutical solution.

Preferably, the pH of the solution is from 1.5 to 2.5.

According to a preferred embodiment, the oral pharmaceutical solution according to the invention comprises from 2 mg/ml to 10 mg/ml of lenalidomide or a pharmaceutically acceptable salt thereof, and a pharmaceutically acceptable carrier comprising
a) a glycol selected from propylene glycol or polyethylene glycol having an average molecular weight from 150 to 1500, wherein the glycol concentration is from 200 mg/ml to 600 mg/ml,
b) a polyol selected from glycerol, sorbitol, mannitol, maltitol, xylitol, erythritol, isomalt, lactitol, polyvinyl alcohol, or mixtures thereof, wherein the polyol concentration is from 50 mg/ml to 400 mg/ml,
c) water,
wherein the pH of the solution is from 1.0 to 3.0.

According to another preferred embodiment, the oral pharmaceutical solution according to the invention comprises from 2 mg/ml to 10 mg/ml of lenalidomide or a pharmaceutically acceptable salt thereof, and a pharmaceutically acceptable carrier comprising
a) propylene glycol or polyethylene glycol having an average molecular weight from 150 to 1500, wherein the glycol concentration is from 200 mg/ml to 600 mg/ml,
b) a polyol selected from maltitol, glycerol, mannitol, sorbitol or xylitol, wherein the polyol concentration is from 50 mg/ml to 400 mg/ml,
c) water,
wherein the pH of the solution is from 1.0 to 3.0.

Preferably, the oral pharmaceutical solution according to the invention is free of surfactants.

The oral pharmaceutical solution according to the invention may also optionally contain additional excipients commonly used in preparing oral liquid compositions, such as an antimicrobial preservative, an antioxidant, a viscosity adjusting agent, a flavouring agent or a sweetener.

Examples of antimicrobial preservatives as referred to herein include but are not limited to sodium benzoate, benzoic acid, boric acid, sorbic acid and salts thereof, benzyl alcohol, parahydroxy benzoic acids and their alkyl esters, methyl, ethyl and propyl parahydroxy benzoates or their salts, or mixtures thereof.

Examples of antioxidants as referred to herein include but are not limited to sodium metabisulfite, butylated hydroxyanisole, butylated hydroxytoluene, ethylenediamine tetraacetic acid, ascorbic acid, α-tocopherol, propyl gallate, or mixtures thereof.

Examples of flavouring agents as referred to herein include but are not limited to fruit flavours such as orange, banana, strawberry, cherry, wild cherry, lemon and the like and other flavourings, such as cardamom, anise, mint, menthol, vanillin, bubble gum, or mixtures thereof.

Examples of sweeteners as referred to herein include but are not limited to sugars, i.e. carbohydrates, such as sucrose, glucose, dextrose, lactose, fructose and galactose as well as other sweetening agents known in the art such as sucralose, aspartame, acesulfame-K, thaumatin, mogroside, saccharin and salts thereof, sodium cyclamate, erythritol, glycyrrhizin, monosodium glycyrrhizinate, monoamonium glycyrrhizinate, or mixtures thereof.

The oral pharmaceutical solution according to the invention is preferably supplied as a multidose preparation. Each dose from a multidose container, such as an amber type III glass 50 or 100 ml bottle sealed with a suitable seal, such as a child resistant, tamper evident screw cap, can be administered by means of a device suitable for measuring the prescribed volume. The device is usually a spoon or a cup for volumes of 5 ml or multiples thereof, or an oral syringe for other volumes.

The oral pharmaceutical solution according to the invention exhibits acceptable stability when stored under conditions of room temperature (25°C) and relative humidity (RH) of 60%. The stability of the solution increases when it is stored at lower temperatures (4 - 8°C). Importantly, this increased stability is maintained even when the storage temperature increases from 4 - 8°C to 25°C for several days.

The oral pharmaceutical solution of the present invention may be prepared using methods well known in the art and using regular manufacturing equipment.

For example, it may be prepared using the following process:
The active substance (lenalidomide or pharmaceutically acceptable salt of lenalidomide) and the excipients are weighed. Purified water is added to a vessel. The glycol(s) and polyol(s) are successively added to the vessel. The active substance is then added under continuous stirring. Hydrochloric acid solution (5N) is added under continuous stirring until the active substance is completely dissolved. An aqueous buffer solution, if present, is prepared in a separate vessel, and is added under continuous stirring until the active substance is completely dissolved. Preservative, if present, is also added under continuous stirring until complete dissolution. Flavor and the remaining excipients, if present, are successively added under continuous stirring, until complete dissolution. The pH of the solution is adjusted to the desired value by adding, for example, aqueous sodium hydroxide or hydrochloric acid. Finally, the volume is adjusted with purified water or with the buffer solution.

### EXAMPLES

The following examples show the influence of the proposed, according to the invention, aqueous carrier, on the solubility and stability of lenalidomide.

### EXAMPLE 1

Purified water was added to a vessel. Povidone, the glycols (if present) and polyol (if present) were successively added to the vessel. Lenalidomide was then added under continuous stirring. An aqueous buffer solution (citric acid monohydrate/sodium citrate dihydrate/hydrochloric acid), was prepared in a different vessel and was added under continuous stirring until lenalidomide was completely dissolved. Ascorbic acid, sucralose and the flavor were also added under continuous stirring until complete dissolution. The pH of the solution was adjusted to the desired value by adding aqueous sodium hydroxide (1N) or hydrochloric acid solution (5N). Finally, the volume was adjusted with purified water. The compositions were filled in amber type III glass 50 ml bottles.

The compositions of the prepared oral solutions are shown in Table 1.

**Table 1**

| **Component** | **mg/ml** | | | | |
|---|---|---|---|---|---|
| | **Trial 0** | **Trial 1** | **Trial 2** | **Trial 3** | **Trial I** |
| Lenalidomide | 5 | 5 | 5 | 5 | 5 |
| Povidone (Kollidon 90F) | 10 | 5 | 10 | 10 | 10 |
| Sucralose | 5 | 5 | 5 | 5 | 5 |
| PG | - | 250 | 250 | 300 | 250 |
| PEG-400 | - | 200 | 300 | 300 | 200 |
| PEG-1450 | - | 50 | 50 | 50 | 50 |
| Glycerol | - | - | - | - | 50 |
| Ascorbic acid | 1.0 | 1.0 | 1.0 | 1.0 | 1.0 |
| Orange flavor | 1.0 | 1.0 | 1.0 | 1.0 | 1.0 |
| Buffer solution (citric acid monohydrate/sodium citrate dihydrate/hydrochloric acid) | Qs | | | | |
| NαOH (1N) / HCL (5N) QS to | pH 2.0 | pH 2.0 | pH 2.2 | pH 2.0 | pH 2.0 |
| Purified Water | QS to 1mL | | | | |

| | | | | | |
|---|---|---|---|---|---|
| PG; Propylene glycol, PEG; Polyethylene glycol | | | | | |

Attempts to prepare an oral solution by combining lenalidomide with povidone as cosolvent (trial 0) led to the formation of precipitate within minutes of the preparation of the composition. Thus, the composition of Trial 0 was not subjected to the stability study.

Storage conditions of room temperature (25°C) and relative humidity (RH) of 60% were applied for a period of two months to samples prepared in Trials 1, 2, 3 & I. Quantification of lenalidomide and its impurities, in the prepared solution, was performed by HPLC. The results are shown in Table 2.

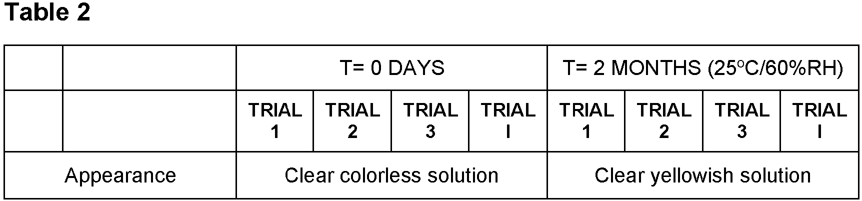

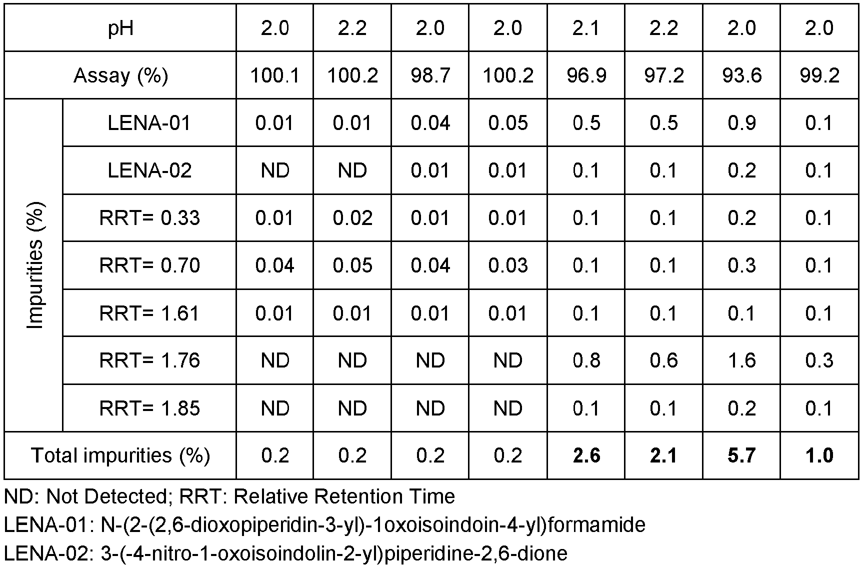

Surprisingly it was found that solutions of trials 1, 2 & 3 containing only glycols as cosolvents, even at high concentrations, are less stable than the solution of trial I, which in addition to glycols it contains glycerol (a polyol) as cosolvent.

### EXAMPLE 2

Purified water was added to a vessel. The glycols and polyols were successively added to the vessel. Lenalidomide was then added under continuous stirring. Hydrochloric acid solution (5N) was added under continuous stirring until lenalidomide was completely dissolved. Sucralose and the flavor were also added under continuous stirring until complete dissolution. The pH of the solution was adjusted by adding aqueous sodium hydroxide (1N) or hydrochloric acid solution (5N) to the desired value. Finally, the volume was adjusted with purified water. The compositions were filled in amber type III glass 50 ml bottles.

The compositions of the prepared oral solutions are shown in Table 3.

**Table 3**

| **Component** | **mg/ml** | | | |
|---|---|---|---|---|
| | **Trial II** | **Trial III** | **Trial IV** | **Trial V** |
| Lenalidomide | 5 | 5 | 5 | 5 |
| Glycerol | 200 | 50 | - | - |
| Sorbitol | - | 150 | - | - |
| Maltitol | - | - | 200 | 50 |
| Mannitol | - | - | - | 100 |
| Sucralose | 5 | 5 | 5 | 5 |
| Propylene glycol | 150 | 150 | 200 | 200 |
| PEG-400 | 100 | 200 | 200 | 200 |
| PEG-1450 | 50 | 50 | 50 | 100 |
| Orange flavor | 2.0 | 2.0 | 2.0 | 2.0 |
| HCL (5N) | Qs | | | |
| NαOH (1N) / HCL (5N) QS to | pH 2.0 | pH 2.4 | pH 2.0 | pH 2.0 |
| Purified Water | QS to 1mL | | | |

Storage conditions of room temperature (25°C) and relative humidity of 60% were applied for a period of four months. Quantification of lenalidomide and its impurities, in the prepared solutions, was performed by HPLC. The results are shown in Table 4.

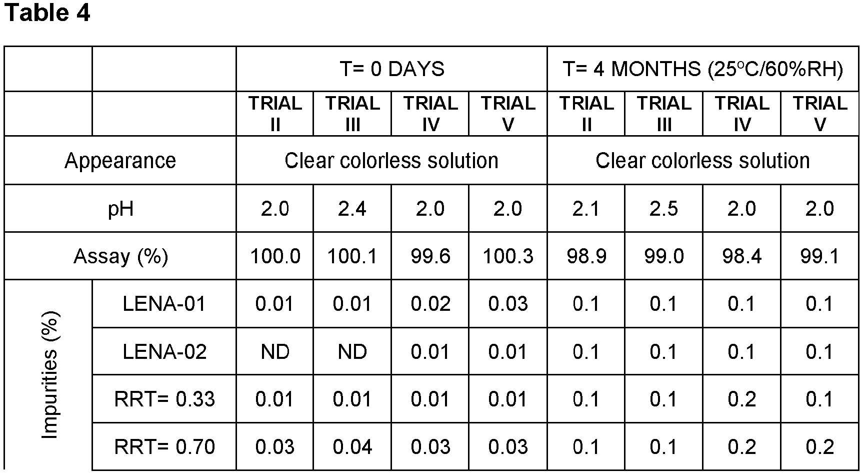

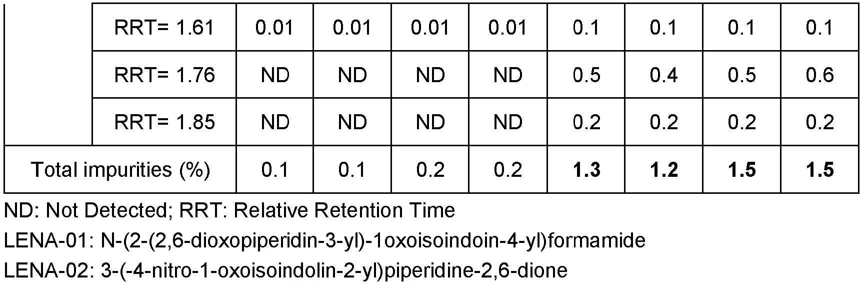

From this study, it is further corroborated that in the presence of at least one polyol and at least one glycol, the stability of lenalidomide is greatly enhanced since certain individual unknown impurities (as well as total impurities) in trials II to V are present at lower levels after a storage period of four months in comparison to the impurities observed in trials 1, 2 & 3 (of example 1) after a storage period of only two months, under the same conditions (25°C/60%RH).

### EXAMPLE 3

Trials VI & VII (compositions without buffer); Purified water was added to a vessel. The glycols and polyols were successively added to the vessel. Lenalidomide was then added under continuous stirring. Hydrochloric acid solution (5N) was added under continuous stirring until lenalidomide was completely dissolved. Sucralose and the flavor were also added under continuous stirring until complete dissolution. The pH of the solution was adjusted by adding aqueous sodium hydroxide (1N) or hydrochloric acid solution (5N) to the desired value. Finally, the volume was adjusted with purified water.

Trials VIII & IX (compositions comprising buffer); Purified water was added to a vessel. The glycols and polyols were successively added to the vessel. Lenalidomide was then added under continuous stirring. An aqueous buffer solution (citric acid monohydrate /sodium citrate dihydrate/hydrochloric acid), was prepared in a different vessel and was added under continuous stirring until lenalidomide is completely dissolved. Sucralose and the flavor were also added under continuous stirring until complete dissolution. The pH of the solution was adjusted by adding aqueous sodium hydroxide (1N) or hydrochloric acid solution (5N) to the desired value. Finally, the volume was adjusted with purified water. The compositions were filled in amber type III glass 50 ml bottles.

The compositions of the prepared solutions are shown in Tables 5a & 5b.

**Table 5a; Compositions prepared without buffer solution**

| **Component** | **mg/ml** | |
|---|---|---|
| | **Trial VI** | **Trial VII** |
| Lenalidomide | 5 | 5 |
| Glycerol | 200 | 100 |
| Sorbitol | - | 100 |
| Sucralose | 5 | 5 |
| Propylene glycol | 250 | - |
| PEG-400 | - | 300 |
| PEG-1450 | 50 | - |
| Orange flavor | 2.0 | 2.0 |
| HCL (5N) | Qs | |
| NαOH (1N) / HCL (5N) QS to | pH 2.0 | pH 2.4 |
| Purified Water | QS to 1mL | |

**Table 5b; Compositions prepared with buffer solution**

| **Component** | **mg/ml** | |
|---|---|---|
| | **Trial VIII** | **Trial IX** |
| Lenalidomide | 5 | 5 |
| Maltitol | 200 | 50 |
| Mannitol | - | 50 |
| Sucralose | 5 | 5 |
| Propylene glycol | 300 | 200 |
| PEG-400 | - | 200 |
| PEG-1450 | 50 | - |
| Orange flavor | 2.0 | 2.0 |
| Buffer (citric acid monohydrate/ sodium citrate dihydrate/hydrochloric acid) | Qs | |
| NαOH (1N) / HCL (5N) QS to | pH 2.0 | pH 2.0 |
| Purified Water | QS to 1mL | |

Storage conditions of room temperature (25°C) and relative humidity of 60% were applied for a period of four months. Quantification of lenalidomide and its impurities, in the compositions prepared, was performed by HPLC. The results are shown in Table 6.

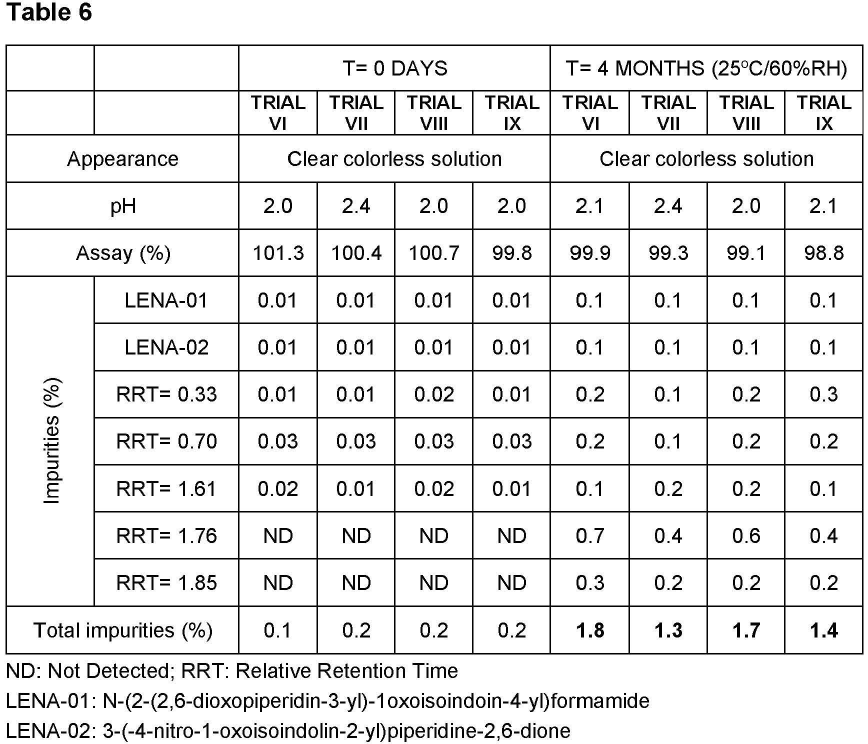

From this study, it is further corroborated that in the presence of at least one polyol and at least one glycol, the stability of lenalidomide is greatly enhanced, since certain individual unknown impurities (as well as total impurities) in trials VI to IX are present at lower levels after a storage period of four months, in comparison to the impurities observed in trials 1, 2 & 3 (of example 1) after a storage period of only two months under the same conditions (25°C/60%RH).

### EXAMPLE 4

Purified water was added to a vessel. Propylene glycol (if present) and glycerol (if present) were successively added to the vessel. Lenalidomide was then added under continuous stirring. An aqueous buffer solution (citric acid monohydrate/sodium citrate dihydrate/hydrochloric acid), was prepared in a different vessel and was added under continuous stirring until lenalidomide was completely dissolved. Sucralose and the flavor were also added under continuous stirring until complete dissolution. The pH of the solution was adjusted to the desired value by adding aqueous sodium hydroxide (1N) or hydrochloric acid solution (5N). Finally, the volume was adjusted with purified water. The compositions were filled in amber type III glass 50 ml bottles.

The compositions of the prepared oral solutions are shown in Table 7.

**Table 7**

| **Component** | **mg/ml** | | | |
|---|---|---|---|---|
| | **Trial 4** | **Trial X** | **Trial XI** | **Trial XII** |
| Lenalidomide | 5 | 5 | 5 | 5 |
| Sucralose | 5 | 5 | 5 | 5 |
| PG | - | 200 | - | 100 |
| Glycerol | - | - | 200 | 100 |
| Orange flavor | 1.0 | 1.0 | 1.0 | 1.0 |
| Buffer (citric acid monohydrate/ sodium citrate dihydrate/hydrochloric acid) | Qs | | | |
| NαOH (1N) / HCL (5N) QS to | pH 2.0 | pH 2.0 | pH 2.0 | pH 2.0 |
| Purified Water | QS to 1mL | | | |

| | | | | |
|---|---|---|---|---|
| PG; Propylene glycol, PEG; Polyethylene glycol | | | | |

Attempts to prepare an oral solution, without a cosolvent, by dissolving lenalidomide, in an aqueous buffer at pH 2.0 (trial 4) led to the formation of precipitate within about four days after the preparation of the composition, even when the composition was stored in the refrigerator. The composition of trial 4 was not subjected to the stability study.

Storage conditions of temperature 4 - 8°C were applied for a period of 180 days and of temperature 25°C for 5 days (samples were left on the lab bench for one day every 30 days of storage period) to samples of trials X, XI & XII. Quantification of lenalidomide and its impurities, in the prepared solution, was performed by HPLC. The results are shown in Table 8.

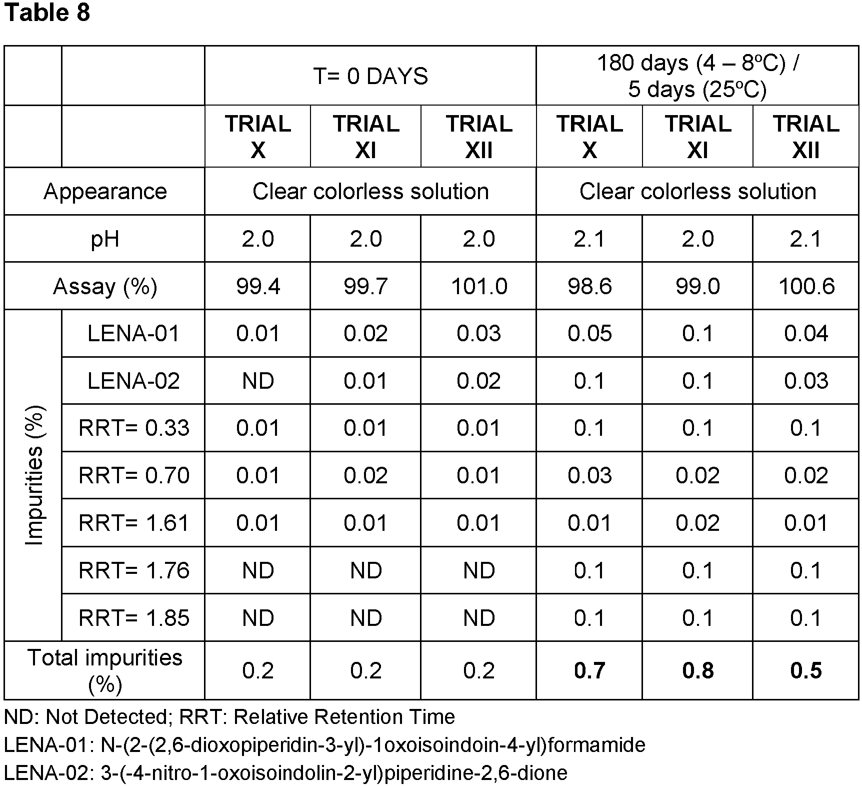

Surprisingly it was found that solutions of trial XII containing a polyol and a glycol as cosolvents presents better stability in comparison to solutions of trials X & XI containing only a glycol or only a polyol when stored in a refrigerator, even when the storage temperature increases to 25°C for a considerable period.

## Claims

1. An oral pharmaceutical solution comprising lenalidomide or a pharmaceutically acceptable salt thereof as active ingredient and a pharmaceutically acceptable carrier comprising
a) a glycol selected from propylene glycol, polyethylene glycol having an average molecular weight from 150 to 1500, or mixtures thereof,
b) a polyol selected from glycerol, sorbitol, mannitol, maltitol, xylitol, erythritol, isomalt, lactitol, polyvinyl alcohol, or mixtures thereof,
c) water,
wherein the pH of the solution is from 1.0 to 3.0.

2. The oral pharmaceutical solution according to claim 1, wherein the glycol is selected from propylene glycol, polyethylene glycol 200, polyethylene glycol 300, polyethylene glycol 400, polyethylene glycol 600, polyethylene glycol 800 or polyethylene glycol 1450.

3. The oral pharmaceutical solution according to any one of the preceding claims, wherein the polyol is selected from glycerol, sorbitol, mannitol, maltitol, xylitol, or mixtures thereof.

4. The oral pharmaceutical solution according to any one of the preceding claims, wherein the concentration of lenalidomide or the pharmaceutically acceptable lenalidomide salt in the solution is from 2 mg/ml to 10 mg/ml.

5. The oral pharmaceutical solution according to any one of the preceding claims, wherein the glycol concentration in the solution is from 200 to 600 mg/ml.

6. The oral pharmaceutical solution according to any one of the preceding claims, wherein the glycol concentration in the solution is from 250 to 550 mg/ml.

7. The oral pharmaceutical solution according to any one of the preceding claims, wherein the glycol concentration in the solution is from 300 to 500 mg/ml.

8. The oral pharmaceutical solution according to any one of the preceding claims, wherein the polyol concentration in the solution is from 50 mg/ml to 400 mg/ml.

9. The oral pharmaceutical solution according to any one of the preceding claims, wherein the polyol concentration in the solution is from 100 mg/ml to 350 mg/ml.

10. The oral pharmaceutical solution according to any one of the preceding claims, wherein the polyol concentration in the solution is from 100 mg/ml to 300 mg/ml.

11. The oral pharmaceutical solution according to any one of the preceding claims, wherein the pH of the solution is from 1.5 to 2.5.

12. The oral pharmaceutical solution according to any one of the preceding claims, wherein the active ingredient is lenalidomide.

13. The oral pharmaceutical solution according to any one of the preceding claims, wherein the solution does not comprise a surfactant.

## Patentansprüche

1. Orale pharmazeutische Lösung, umfassend Lenalidomid oder ein pharmazeutisch akzeptables Salz davon als Wirkstoff und einen pharmazeutisch akzeptablen Trägerstoff, umfassend
a) ein Glykol, ausgewählt aus Propylenglykol, Polyethylenglykol, das eine mittlere Molekülmasse von 150 bis 1500 aufweist, oder Mischungen davon,
b) ein Polyol, ausgewählt aus Glycerin, Sorbit, Mannit, Maltit, Xylit, Erythrit, Isomalt, Lactit, Polyvinylalkohol oder Mischungen davon,
c) Wasser,
wobei der pH-Wert der Lösung von 1,0 bis 3,0 beträgt.

2. Orale pharmazeutische Lösung nach Anspruch 1, wobei das Glykol aus Propylenglykol, Polyethylenglykol 200, Polyethylenglykol 300, Polyethylenglykol 400, Polyethylenglykol 600, Polyethylenglykol 800 oder Polyethylenglykol 1450 ausgewählt ist.

3. Orale pharmazeutische Lösung nach einem der vorhergehenden Ansprüche, wobei das Polyol aus Glycerin, Sorbit, Mannit, Maltit, Xylit oder Mischungen davon ausgewählt ist.

4. Orale pharmazeutische Lösung nach einem der vorhergehenden Ansprüche, wobei die Konzentration von Lenalidomid oder des pharmazeutisch akzeptablen Lenalidomidsalzes in der Lösung von 2 mg/ml bis 10 mg/ml beträgt.

5. Orale pharmazeutische Lösung nach einem der vorhergehenden Ansprüche, wobei die Glykolkonzentration in der Lösung von 200 bis 600 mg/ml beträgt.

6. Orale pharmazeutische Lösung nach einem der vorhergehenden Ansprüche, wobei die Glykolkonzentration in der Lösung von 250 bis 550 mg/ml beträgt.

7. Orale pharmazeutische Lösung nach einem der vorhergehenden Ansprüche, wobei die Glykolkonzentration in der Lösung von 300 bis 500 mg/ml beträgt.

8. Orale pharmazeutische Lösung nach einem der vorhergehenden Ansprüche, wobei die Polyolkonzentration in der Lösung von 50 mg/ml bis 400 mg/ml beträgt.

9. Orale pharmazeutische Lösung nach einem der vorhergehenden Ansprüche, wobei die Polyolkonzentration in der Lösung von 100 mg/ml bis 350 mg/ml beträgt.

10. Orale pharmazeutische Lösung nach einem der vorhergehenden Ansprüche, wobei die Polyolkonzentration in der Lösung von 100 mg/ml bis 300 mg/ml beträgt.

11. Orale pharmazeutische Lösung nach einem der vorhergehenden Ansprüche, wobei der pH-Wert der Lösung von 1,5 bis 2,5 beträgt.

12. Orale pharmazeutische Lösung nach einem der vorhergehenden Ansprüche, wobei der Wirkstoff Lenalidomid ist.

13. Orale pharmazeutische Lösung nach einem der vorhergehenden Ansprüche, wobei die Lösung kein Tensid umfasst.

## Revendications

1. Solution pharmaceutique orale comprenant du lénalidomide ou un de ses sels pharmaceutiquement acceptables comme ingrédient actif et un véhicule pharmaceutiquement acceptable comprenant
a) un glycol choisi parmi le propylène glycol, le polyéthylène glycol ayant un poids moléculaire moyen de 150 à 1 500 ou leurs mélanges,
b) un polyol choisi parmi le glycérol, le sorbitol, le mannitol, le maltitol, le xylitol, l'érythritol, l'isomalt, le lactitol, l'alcool polyvinylique ou leurs mélanges,
c) de l'eau,
le pH de la solution étant de 1,0 à 3,0.

2. Solution pharmaceutique orale selon la revendication 1, dans laquelle le glycol est choisi parmi le propylène glycol, le polyéthylène glycol 200, le polyéthylène glycol 300, le polyéthylène glycol 400, le polyéthylène glycol 600, le polyéthylène glycol 800 ou le polyéthylène glycol 1450.

3. Solution pharmaceutique orale selon l'une quelconque des revendications précédentes, dans laquelle le polyol est choisi parmi le glycérol, le sorbitol, le mannitol, le maltitol, le xylitol ou leurs mélanges.

4. Solution pharmaceutique orale selon l'une quelconque des revendications précédentes, dans laquelle la concentration de lénalidomide ou du sel de lénalidomide pharmaceutiquement acceptable dans la solution est de 2 mg/ml à 10 mg/ml.

5. Solution pharmaceutique orale selon l'une quelconque des revendications précédentes, dans laquelle la concentration de glycol dans la solution est de 200 à 600 mg/ml.

6. Solution pharmaceutique orale selon l'une quelconque des revendications précédentes, dans laquelle la concentration de glycol dans la solution est de 250 à 550 mg/ml.

7. Solution pharmaceutique orale selon l'une quelconque des revendications précédentes, dans laquelle la concentration de glycol dans la solution est de 300 à 500 mg/ml.

8. Solution pharmaceutique orale selon l'une quelconque des revendications précédentes, dans laquelle la concentration de polyol dans la solution est de 50 mg/ml à 400 mg/ml.

9. Solution pharmaceutique orale selon l'une quelconque des revendications précédentes, dans laquelle la concentration de polyol dans la solution est de 100 mg/ml à 350 mg/ml.

10. Solution pharmaceutique orale selon l'une quelconque des revendications précédentes, dans laquelle la concentration de polyol dans la solution est de 100 mg/ml à 300 mg/ml.

11. Solution pharmaceutique orale selon l'une quelconque des revendications précédentes, dans laquelle le pH de la solution est de 1,5 à 2,5.

12. Solution pharmaceutique orale selon l'une quelconque des revendications précédentes, dans laquelle l'ingrédient actif est le lénalidomide.

13. Solution pharmaceutique orale selon l'une quelconque des revendications précédentes, dans laquelle la solution ne comprend pas de tensioactif.
